# NOUVEAU FASCICULE DE BREVET EUROPEEN

(11) **EP 1 007 030 B3**
(45) Date de publication du présent fascicule: **04.07.2018**
(45) Mention de la délivrance du brevet: 21.04.2004
(21) Numéro de dépôt: 98932234.2
(22) Date de dépôt: 19.06.1998
(51) Int. Cl.: A61K 31/34, A61K 9/20, A61P 9/06

(54) **COMPOSITION PHARMACEUTIQUE SOLIDE CONTENANT DES DERIVES DE BENZOFURANNE**
FESTE ARZNEIZUBEREITUNG ENTHALTEND BENZOFURANDERIVATE
SOLID PHARMACEUTICAL COMPOSITION CONTAINING BENZOFURANE DERIVATIVES

(30) Priorité: 23.06.1997 FR 9707795
(43) Date de publication de la demande: 14.06.2000
(73) Titulaire: Sanofi, 75008 Paris (FR)
(72) Inventeur: ABRAMOVICI, Bernard, F-34990 Juvignac (FR); GAUTIER, Jean-Claude, F-34830 Clapiers (FR); GROMENIL, Jean-Claude, F-34560 Mountbazin (FR); MARRIER, Jean-Marie, F-34970 Lattes (FR)
(74) Mandataire: Bouron, Estelle
(86) Numéro de dépôt international: PCT/FR1998/001285
(87) Numéro de publication internationale: WO 1998/058643

(56) Documents cités:
- WO-A-97/17064
- FR-A- 2 735 978
- US-A- 5 118 707
- CHEMICAL ABSTRACTS, vol. 123, no. 8, 21 août 1995 Columbus, Ohio, US; abstract no. 93077, XP002057969 & R. V. MARTIN-ALGARRA ET AL.: "EFFECTS OF POLYSORBATE 80 ON AMIODARONE INTESTINAL ABSORPTION IN THE RAT" INT. J. PHARM., vol. 122, no. 1,2, 1995, pages 1-8,
- CHEMICAL ABSTRACTS, vol. 121, no. 6, 8 août 1994 Columbus, Ohio, US; abstract no. 65480, XP002057970 & R. V. MARTIN-ALGARRA ET AL.: "EFFECTS OF SURFACTANTS ON AMIODARONE INTESTINAL ABSORPTION.I.SODIUM LAURYL SULFATE." PHARM. RES., vol. 11, no. 7, 1994, pages 1042-1047,
- CHEMICAL ABSTRACTS, vol. 71, no. 26, 29 décembre 1969 Columbus, Ohio, US; abstract no. 128658, XP002057971 & J. L. RAVIN ET AL.: "EFFECT OF POLYSORBATE 80 ON THE SOLUBILITY AND IN VIVO AVAILABILITY OF 2-BUTYL-3-BENZOFURANYL 4-[2-(DIETHYLAMINO)ETHOXY]-3,5-DIIODOPHENY L KETONE HYDROCHLORIDE (SKF 33134 A)." J. PHARM. SCI., vol. 58, no. 10, 1969, pages 1242-1245,

## Description

La présente invention se rapporte, d'une manière générale, à une nouvelle composition pharmaceutique pour administration orale contenant comme principe actif un dérivé de benzofuranne.

Plus précisément, l'invention concerne une composition pharmaceutique solide pour administration orale contenant comme principe actif, un dérivé de benzofuranne à activité antiarythmique.

Par "dérivé de benzofuranne à activité antiarythmique", on désigne dans le cadre de la présente invention, un composé benzofurannique choisi parmi ceux décrits dans les brevets US 3248401, US 5223510 et EP 338746 ainsi que dans les demandes de brevet WO 88/07996, WO 89/02892, WO 90/02743 et WO 94/29289.

De l'ensemble de ces composés, on peut citer, de manière préférentielle, le 2-n-butyl-3-[4-(3-di-n-butylaminopropoxy)benzoyl]-5-méthylsulfonamidobenzofuranne ou dronédarone et ses sels pharmaceutiquement acceptables décrits dans le brevet US 5223510 de même que le 2-n-butyl-3-(3,5-diiodo-4-diéthylaminoéthoxy-benzoyl) benzofuranne ou amiodarone et ses sels pharmaceutiquement acceptables décrits dans le brevet US 3248401.

De même, par "composition pharmaceutique solide", on entend essentiellement une composition pharmaceutique formée en totalité d'ingrédients solides pulvérulents et compressibles à la température ambiante, comprenant le principe actif et les excipients, ces ingrédients étant essentiellement sous forme de poudre.

Par conséquent, les compositions pharmaceutiques dites semi-solides, formées de substances se présentant sous forme pâteuse ou cireuse lorsqu'elles sont portées à température modérée (< 70°C), ne font pas partie de l'invention.

Les composés antiarythmiques utilisés dans le cadre de l'invention, notamment la dronédarone et l'amiodarone sous forme de leur chlorhydrate, sont caractérisés par une faible solubilité en milieu aqueux.

A titre d'exemple, la courbe de solubilité du chlorhydrate de dronédarone à température ambiante et en fonction du pH révèle une solubilité maximale vers les pH de 3 à 5, d'environ 1 à 2mg/ml mais très faible à des pH de l'ordre de 6 à 7 puisqu'elle n'est plus que 10 µg/ml à pH = 7.

Quant au chlorhydrate d'amiodarone, sa solubilité est, à température ambiante, de 0,3 à 0,9 mg/ml dans la gamme de pH de 3 à 4 et de quelques µg/ml à pH = 7.

Ainsi, il est possible de dissoudre 400mg de chlorhydrate de dronédarone dans 200 ml de milieu aqueux tamponné à pH = 4 (solution aqueuse 0,1M en NaH₂PO₄).

Par contre, dans ce milieu dilué au 1/10 par une solution aqueuse tamponnée à pH = 7 (solution aqueuse 0,1M en Na₂HPO₄), le chlorhydrate de dronédarone précipite (pH du milieu final : 6,7).

Ces conditions de solubilité étant semblables à celles enregistrées dans le tractus gastro-intestinal, on peut supposer que le chlorhydrate de dronédarone risque d'être soumis, dans l'estomac, à des conditions acides favorables à sa solubilisation mais de rencontrer, au contraire, un milieu de pH = 6 à 7 dès son entrée dans l'intestin, c'est-à-dire un milieu non solubilisant dans lequel il va précipiter.

Ce comportement en milieu intestinal permet probablement d'expliquer in vivo, la faible biodisponibilité du chlorhydrate de dronédarone et les différences observées après administration en présence ou non de nourriture.

En fait, on a remarqué que la biodisponibilité du chlorhydrate de dronédarone chez le chien comme chez l'homme est exaltée après la prise de nourriture, en particulier des graisses, qui peut modifier fortement la cinétique de précipitation de ce principe actif et aussi favoriser sa mise en émulsion.

Comme l'absorption de nourriture provoque la sécrétion de sels biliaires, qui sont des agents tensioactifs anioniques, celle-ci pourrait semble-t-il influencer de manière favorable la solubilisation du chlorhydrate de dronédarone.

Toutefois, des tests effectués à cet effet, ont montré au contraire que ce principe actif précipite en présence de sels biliaires tel que le taurocholate de sodium.

La mise au point d'une composition pharmaceutique orale de dronédarone, d'amiodarone ou de leurs sels pharmaceutiquement acceptables, capable d'éviter la précipitation du principe actif en milieu neutre et de réduire la variabilité d'absorption plasmatique de ce principe actif, c'est-à-dire d'amener une biodisponibilité acceptable indépendamment de la présence de nourriture, reste d'un intérêt essentiel.

Or, on a maintenant trouvé de manière surprenante que l'association d'un agent tensioactif hydrophile non ionique à la dronédarone, l'amiodarone ou à leurs sels pharmaceutiquement acceptables, permet de maintenir la solubilisation de ce principe actif en milieu neutre et de réduire, chez l'homme, sa variabilité d'absorption dans le sang.

Cette observation est d'autant plus surprenante que des tests préliminaires effectués sur chien n'ont pas permis de montrer qu'un agent tensioactif hydrophile non ionique était capable d'augmenter la biodisponibilité à jeun du chlorhydrate de dronédarone, et par la même occasion, de réduire la variabilité d'absorption plasmatique de ce principe actif.

Ainsi, l'invention se rapporte à une composition pharmaceutique solide pour administration orale comprenant un dérivé de benzofuranne à activité antiarythmique ou un de ses sels pharmaceutiquement acceptables, comme principe actif, et un agent tensioactif hydrophile non ionique pharmaceutiquement acceptable éventuellement en association avec un ou plusieurs excipients pharmaceutiques.

Cette composition pharmaceutique peut se présenter sous toute forme pharmaceutique solide convenant à l'administration orale telle que comprimé sécable ou non, granule, gélule, poudre en sachet unitaire.

En conséquence, un autre objet de l'invention se rapporte à la composition pharmaceutique orale ci-dessus sous forme de comprimé, granule, gélule ou poudre.

L'agent tensioactif hydrophile non ionique utilisé dans la composition de l'invention peut être choisi parmi :
- des copolymères oxyde d'éthylène/oxyde de propylène ci-après dénommés poloxamères tels que le poloxamère 124 commercialisé sous la marque SYNPERONIC® PE/L44; le poloxamère 188 commercialisé sous la marque PLURONIC® F68 ou SYNPERONIC® PE/F68; le poloxamère 237 commercialisé sous la marque PLURONIC® F87 ou SYNPERONIC® PE/F87; le poloxamère 338 commercialisé sous la marque SYNPERONIC® PE/F108 ou le poloxamère 407 commercialisé sous la marque PLURONIC® F127, SYNPERONIC® PE/F127 ou LUTROL® F127.
- des huiles de ricin polyéthoxylées telles que celles commercialisées sous la marque CREMOPHOR® RH40.
- des polysorbates éthoxylés tels que les polysorbate 20, polysorbate 40, polysorbate 60 et polysorbate 80 commercialisés respectivement sous les marques TWEEN® 20, TWEEN® 40, TWEEN® 60, et TWEEN® 80.
- ou encore des hydroxystéarates de polyéthylène tels que l'hydroxystéarate de polyéthylène 660 commercialisé sous la marque SOLUTOL® HS15.
A titre d'agent tensioactif préféré, on peut citer le poloxamère 407.

Habituellement, le tensioactif hydrophile non ionique en question est incorporé dans les compositions solides de l'invention à raison de 1% à 50% en poids par rapport au principe actif sous forme de base quelque soit la forme pharmaceutique, unitaire ou non, adoptée pour leur conditionnement

Pour la préparation de compositions solides sous forme de comprimé ou conditionnées sous forme de gélule, on utilisera par exemple de 1% à 20% en poids de tensioactif par rapport au principe actif sous forme de base, de préférence de 5% à 15%.

A titre indicatif mais non limitatif, la quantité de principe actif peut varier de 50 à 500mg par unité d'administration sous forme de comprimé, ce qui entraîne l'incorporation d'une quantité d'agent tensioactif entre 0,5 et 100mg. Ces quantités d'agent tensioactif se révèlent parfaitement acceptables avec des formes pharmaceutiques telles que comprimé ou gélule dont les tailles resteront compatibles avec une administration orale.

A titre préférentiel, des compositions pharmaceutiques solides de l'invention par exemple sous forme de comprimé ou de gélule peuvent contenir de 200 à 400mg de principe actif calculés sous forme de base et de 5% à 15% plus particulièrement 10% en poids de tensioactif hydrophile non ionique par rapport au principe actif sous forme de base.

Pour un conditionnement sous forme de poudre en sachet unitaire, on pourra utiliser de 1% à 50% en poids d'agent tensioactif hydrophile non ionique par rapport au principe actif sous forme de base.

Outre, le tensioactif en question, les compositions sous forme solide, selon l'invention, comprendront d'autres excipients pharmaceutiques généralement utilisés dans l'élaboration de formes pharmaceutiques orales.

Ces substances sont parfaitement connues de l'homme du métier qui pourra aisément les sélectionner selon le type de composition orale choisi.

A titre d'exemples non limitatifs, on peut citer des liants généralement des dérivés cellulosiques comme la méthylcellulose, l'hydroxyéthylcellulose, la méthylhydroxypropylcellulose ou encore des macrogols tels que le macrogol 6000; des agents d'écoulement tels que la silice colloïdale; des polymères ou copolymères de vinylpyrrolidone tels que la polyvinylpyrrolidone; des diluants tels que le lactose ou le mannitol; des amidons tels que l'amidon de blé ou de maïs; des lubrifiants tels que le stéarate de magnésium ou le stéaryl fumarate de sodium.

Les compositions de l'invention peuvent être préparées par mise en oeuvre de procédés connus comprenant notamment des techniques de granulation par voie sèche ou humide, par fusion ou par compression directe pour la formation de comprimés.

Par exemple, on peut préparer des comprimés par granulation humide en mélangeant au stade initial l'ensemble des ingrédients y compris le principe actif et l'agent tensioactif, à l'exception toutefois du lubrifiant.

On réalise ensuite des opérations de mouillage avec de l'eau purifiée, séchage et calibrage du grain obtenu, lubrification et compression ou remplissage direct de gélules.

Selon des variantes de cette méthode :
a) on mélange au stade initial l'ensemble des ingrédients y compris le principe actif à l'exception du tensioactif et du lubrifiant et on poursuit par des opérations de mouillage avec une solution aqueuse du tensioactif, granulation, séchage, calibrage, lubrification et compression ou remplissage direct de gélules.
   ou
b) on mélange au stade initial l'ensemble des ingrédients y compris le principe actif et le tensioactif à l'exception du liant et du lubrifiant et on poursuit par la suite par des opérations de mouillage avec une solution aqueuse du liant, granulation, séchage, calibrage, lubrification et compression ou remplissage direct de gélules.

On peut également modifier ces méthodes en incluant un procédé de granulation continu faisant appel à la technique du lit d'air fluidisé au stade de l'opération de mouillage.

En outre, il est possible d'utiliser également un procédé par lequel on mélange au stade initial l'ensemble des ingrédients, à l'exception du lubrifiant, que l'on chauffe à une température de l'ordre de 60°C à 65°C. On réalise alors des opérations de granulation à chaud, calibrage après refroidissement, lubrification et compression ou remplissage direct de gélules.

Selon des techniques de granulation par voie sèche, on mélange d'abord l'ensemble des ingrédients comprenant le principe actif et le tensioactif à l'exception du lubrifiant et on poursuit alors par des opérations de tamisage, compactage, calibrage, lubrification et compression ou remplissage direct de gélules.

Finalement, on peut opérer par compression directe en mettant en oeuvre la suite des opérations suivantes : mélange des ingrédients dont le principe actif et l'agent tensioactif sauf le lubrifiant puis tamisage et mélange, ensuite lubrification et finalement compression ou remplissage direct de gélules.

Les caractéristiques et avantages des compositions orales selon l'invention apparaîtront à la lumière de la description ci-après à partir de compositions orales particulières données à titre d'exemple en référence aux dessins ci-annexés.

### I. Essai de maintien en solution à pH = 6,7

### A. Principe actif seul

On a réalisé des solutions à 2mg/ml de chlorhydrate de dronédarone en milieu tamponné phosphate acide (NaH₂PO₄) à pH=4,5 pendant 2 heures à 37° C en présence ou non de x% de tensioactif hydrophile non ionique à étudier, calculés en poids par rapport au principe actif sous forme de base.

On a ensuite dilué cette solution au 1/10ème dans un milieu phosphate neutre (Na₂HPO₄+NaH₂PO₄), le pH de la solution finale étant de 6,7.

Après 2 heures à 37°C, on a filtré sur filtre de marque ACRODISC® de 5 µm et dosé le principe actif en solution par spectrométrie U.V.

On a ainsi obtenu les résultats suivants :

| Agent tensioactif | x% | % de chlorhydrate de dronédarone en solution |
|---|---|---|
| TWEEN® 20 | 50 | 65 |
| TWEEN® 40 | 50 | 63 |
| TWEEN® 60 | 50 | 74 |
| TWEEN® 80 | 50 | 69 |
| SYNPERONIC® PE/F68 | 50 | 74 |
| SYNPERONIC® PE/F87 | 50 | 75 |
| SYNPERONIC® PE/F127 | 50 | 95 |
| CREMOPHOR® RH 40 | 50 | 64 |
| SOLUTOL® HS 15 | 50 | 59 |
| SYNPERONIC® PE/F127 | 10 | 78 |
| SYNPERONIC® PE/F127 | 5 | 63 |
| - | - | 5 |

### B. Principe actif sous forme de comprimé

On a réalisé des solutions à 2mg/ml de chlorhydrate de dronédarone (exprimé sous forme de base) en milieu tamponné phosphate acide (NaH₂PO₄) à pH = 4,5 ou à 2mg/ml de chlorhydrate d'amiodarone, dans un milieu tamponné à pH = 3,5.

Ces solutions ont été obtenues en mettant à dissoudre des comprimés de chlorhydrate de dronédarone ou de chlorhydrate d'amiodarone contenant ou non 10% de poloxamère 407 (SYNPERONIC® PE/F127), à savoir :

| | Comprimés | |
|---|---|---|
| | α (mg) | A (mg) |
| Chlorhydrate de dronédarone (correspondant à 400mg de base) | 426 | 426 |
| Méthylhydroxypropylcellulose | 12 | 12 |
| Lactose monohydraté | 63,6 | 63,6 |
| Amidon de maïs modifié | 60 | 60 |
| Polyvinylpyrrolidone | 30 | 30 |
| Silice colloïdale anhydre | 2,4 | 2,4 |
| SYNPERONIC® PE/F127 | - | 40 |
| Stéarate de magnésium | 6 | 6 |
| | 600 | 640 |

| | Comprimés | |
|---|---|---|
| | β (mg) | B (mg) |
| Chlorhydrate d'amiodarone | 200 | 200 |
| Lactose monohydraté | 71 | 71 |
| Amidon de maïs modifié | 66 | 66 |
| Polyvinylpyrrolidone réticulée | 6 | 6 |
| Silice colloïdale anhydre | 2,4 | 2,4 |
| SYNPERONIC® PE/F127 | - | 20 |
| Stéarate de magnésium | 4,6 | 4,6 |
| | 350 | 370 |

Après 2 heures de dissolution à 37° C, on dilue ces solutions au 1/10ème dans un milieu phosphate neutre (Na₂HPO₄ + NaH₂PO₄), le pH de la solution finale étant de 6,7.

On a alors poursuivi le test comme décrit au paragraphe A. ci-dessus et on a obtenu les résultats suivants :

| | % de chlorhydrate de dronédarone en solution |
|---|---|
| Comprimé α | 4,6 |
| Comprimé A | 80 |

| | % de chlorhydrate d' amiodarone en solution |
|---|---|
| Comprimé β | 55 |
| Comprimé B | 100 |

Ces résultats montrent que, dans des comprimés, l'incorporation de 10% en poids de poloxamère 407 par rapport à la dronédarone base ou au chlorhydrate d'amiodarone, permet de maintenir en solution de 80% à 100% de principe actif durant 2 heures.

### II. Tests de pharmacocinétique

Des tests comparatifs avec le chlorhydrate de dronédarone ont été effectués sur 16 volontaires de sexe masculin, 8 d'entre eux étant à jeun, les 8 autres non.

Ces essais ont été pratiqués à partir de comprimés de l'invention : l'un à 10% en poids d'agent tensioactif par rapport au poids de dronédarone sous forme de base, (Comprimé A ci-dessus), l'autre à 5% en poids du même tensioactif (Comprimé C ci-dessous) à savoir :

| Comprimé C | mg |
|---|---|
| Chlorhydrate de dronédarone (correspondant à 400mg de base) | 426 |
| Méthylhydroxypropylcellulose | 12 |
| Lactose monohydraté | 63,6 |
| Amidon de maïs modifié | 60 |
| Polyvinylpyrrolidone | 30 |
| Silice colloïdale anhydre | 2,4 |
| SYNPERONIC® PE/F127 | 20 |
| Stéarate de magnésium | 6 |
| | 620 |

comparativement à des compositions dépourvues d'agent tensioactif hydrophile non ionique, c'est-à-dire :
a) comprimé α ci-dessus
b) gélule dotée d'une composition de formulation:

| | mg |
|---|---|
| Chlorhydrate de dronédarone (correspondant à 200mg de base) | 213 |
| Amidon de maïs modifié | 86,2 |
| Lactose monohydraté | 129,2 |
| Talc | 48 |
| Silice colloïdale anhydre | 1,2 |
| Stéarate de magnésium | 2,4 |
| | 480 |

Chacun de ces volontaires a reçu une dose unique de chlorhydrate de dronédarone équivalant à 800mg de base sous forme de gélule ci-dessus, de Comprimé α, de Comprimé A ou de Comprimé C, chaque dose unique étant espacée de la suivante d'une période de 7 jours.

Chez chaque sujet, on a alors effectué des dosages plasmatiques de dronédarone 0, 1, 2, 3, 4, 5, 6, 7, 10, 12, 16 et 24 heures après administration et on a relevé les concentrations maximales de ce principe actif (C max en ng/ml) ainsi que l'aire sous les courbes définies par la concentration du principe actif en fonction du temps (AUC en ng.h/ml).

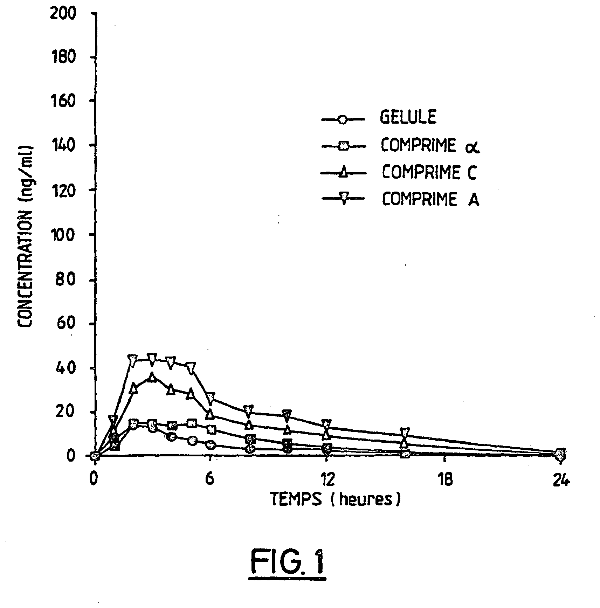

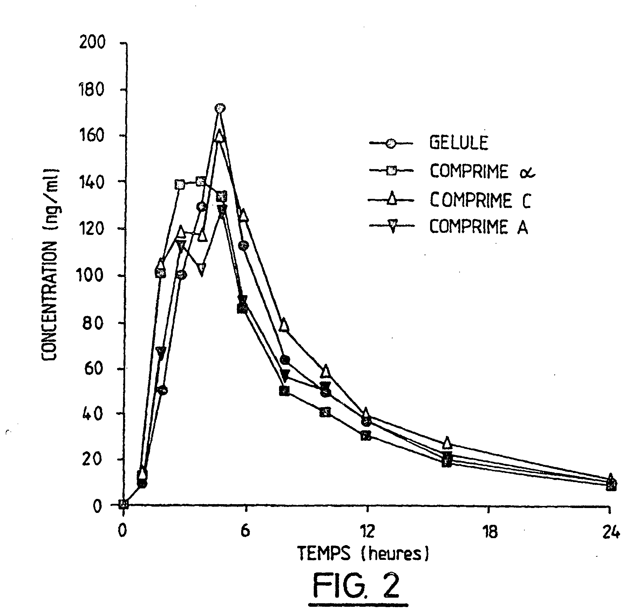

On a répété ce protocole dans une deuxième série d'essais effectués sur les deux mêmes groupes de 8 volontaires alternés, à savoir les 8 volontaires à jeun effectuant le test non à jeun et vice versa.
Les résultats obtenus à jeun sont reproduits à la Figure 1 en annexe et ceux obtenus non à jeun apparaissent à la Figure II en annexe sur lesquelles :
a) la courbe dénommée "gélule" représente la concentration plasmatique moyenne obtenue avec la composition sous forme de gélule.
b) la courbe dénommée "Comprimé α" représente la concentration plasmatique moyenne obtenue avec le Comprimé α
c) la courbe dénommée "Comprimé A" représente la concentration plasmatique moyenne obtenue avec le Comprimé A contenant 10% de tensioactif SYNPERONIC® PE/F127.
d) la courbe dénommée "Comprimé C" représente la concentration plasmatique moyenne obtenue avec le Comprimé C contenant 5% de tensioactif SYNPERONIC® PE/F127.

De ces courbes, on peut notamment :
1) déduire que la présence de l'agent tensioactif exalte la biodisponibilité à jeun du principe actif.
2) dresser les tableaux comparatifs suivants à partir des résultats des C max et AUC obtenus avec chaque formulation chez les volontaires non à jeun par rapport aux résultats correspondants chez les volontaires à jeun, ramenés à 1 :

**TABLEAU I**

| Rapport des C max | Traitement | | | |
|---|---|---|---|---|
| | gélule | Comprimé α | Comprimé C | Comprimé A |
| A jeun | 1 | 1 | 1 | 1 |
| Non à jeun | 12,5 | 10,3 | 4,8 | 2,7 |

**TABLEAU II**

| Rapport des AUC | Traitement | | | |
|---|---|---|---|---|
| | gélule | Comprimé α | Comprimé C | Comprimé A |
| A jeun | 1 | 1 | 1 | 1 |
| Non à jeun | 16,7 | 8,9 | 5,3 | 3,2 |

Ces tableaux montrent que l'agent tensioactif est capable de réduire d'un facteur 2 à 5 les variations de concentrations plasmatiques maximales en principe actif obtenues chez le sujet non à jeun par rapport au sujet à jeun (Tableau I).

De même, on peut conclure que les variations importantes de biodisponibilité enregistrées avec des compositions sans tensioactif ont pu être réduites d'un facteur 1,5 à 5 (Tableau II).

Les Exemples non limitatifs suivants illustrent l'invention.

### EXEMPLE 1

### Comprimé de chlorhydrate de dronédarone

On a préparé des comprimés de chlorhydrate de dronédarone de formulation suivante:

| Ingrédients | mg | % |
|---|---|---|
| Chlorhydrate de dronédarone (correspondant à 400 mg de base) | 426 | 65,5 |
| Méthylhydroxypropylcellulose | 21,1 | 3,25 |
| Lactose monohydraté | 46,55 | 7,2 |
| Amidon de maïs | 45,5 | 7 |
| Polyvinylpyrrolidone | 65 | 10 |
| Poloxamère 407 | 40 | 6,15 |
| Silice colloïdale anhydre | 2,6 | 0,4 |
| Stéarate de magnésium | 3,25 | 0,5 |
| | 650 | 100 |

par application du procédé ci-dessous:

On mélange, après tamisage, 724,2g de chlorhydrate de dronédarone, 35,9g de méthylhydroxypropylcellulose, 79,1g de lactose monohydraté, 77,4g d'amidon de maïs et 82,9g de polyvinylpyrrolidone.

On humecte le mélange avec 68g de poloxamère 407 (SYNPERONIC® PE/F127) en solution dans 408g d'eau purifiée et on granule. On sèche la masse humide à une température de l'ordre de 50°C et on calibre sur tamis de 1,250mm d'ouverture de maille. Au grain ainsi calibré, on mélange 27,6g de polyvinylpyrrolidone, 4,4g de silice colloïdale anhydre et 5,5g de stéarate de magnésium puis on comprime le mélange final à raison de 650mg par unité.

### EXEMPLE 2

### Comprimé de chlorhydrate de dronédarone

On a préparé des comprimés de chlorhydrate de dronédarone de formulation identique à celle de l'Exemple 1 par application du procédé ci-dessous :

On mélange après tamisage, 724,2g de chlorhydrate de dronédarone, 35,9g de méthylhydroxypropylcellulose, 79,1g de lactose monohydraté, 77,4g d'amidon de maïs, 82,9g de polyvinylpyrrolidone et 68g de poloxamère 407 (SYNPERONIC® PE/F127). On humecte ensuite le mélange avec de l'eau purifiée puis on procède de la même manière qu'à l'Exemple 1 pour obtenir des comprimés d'un poids de 650mg par unité.

### EXEMPLE 3

### Comprimé de chlorhydrate de dronédarone

On a préparé des comprimés de chlorhydrate de dronédarone de formulation identique à celle de l'Exemple 1, par application du procédé ci-dessous :

On mélange, après tamisage, 724,2g de chlorhydrate de dronédarone, 79,1g de lactose monohydraté, 77,4 g d'amidon de maïs, 82,9g de polyvinylpyrrolidone et 68g de poloxamère 407 (SYNPERONIC® PE/F127). On humecte le mélange avec 35,9g de méthylhydroxypropylcellulose en solution dans 408g d'eau purifiée et on granule. On sèche la masse humide à une température de l'ordre de 50°C et on calibre sur tamis de 1,250mm d'ouverture de maille. Au grain ainsi calibré, on mélange 27,6g de polyvinylpyrrolidone, 4,4g de silice colloïdale anhydre et 5,5g de stéarate de magnésium puis on comprime le mélange final à raison de 650mg par unité.

### EXEMPLE 4

### Comprimé de chlorhydrate de dronédarone

On a préparé des comprimés de chlorhydrate de dronédarone de formulation suivante :

| Ingrédients | mg | % |
|---|---|---|
| Chlorhydrate de dronédarone (correspondant à 400 mg de base) | 426 | 65,5 |
| Cellulose microaistalline | 65 | 10 |
| Silice colloïdale anhydre | 2,6 | 0,4 |
| Lactose anhydre | 42,65 | 6,6 |
| Polyvinylpyrrolidone | 13 | 2 |
| Poloxamère 407 | 40 | 6,15 |
| Macrogol 6000 | 57,5 | 8,85 |
| Stéarate de magnésium | 3,25 | 0,5 |
| | 650 | 100 |

par mise en oeuvre du procédé ci-dessous:

On mélange, après tamisage, 724,2g de chlorhydrate de dronédarone, 110,5g de cellulose microcristalline, 2,2g de silice colloïdale anhydre, 72,5g de lactose anhydre, 22,1g de polyvinylpyrrolidone, 68g de poloxamère 407 (SYNPERONIC® PE/F127) et 97,8g de macrogol 6000. Sous agitation lente, on porte la température du mélange à 65°C, en cuve thermostatée. On granule ce mélange sous agitation rapide, refroidit jusqu'à température ambiante puis calibre. Au grain calibré, on mélange alors 2,2g de silice colloïdale anhydre et 5,5g de stéarate de magnésium puis on comprime le mélange final à raison de 650mg par unité.

Ce procédé de granulation peut être également réalisé en appareillage à lit d'air fluidisé.

### EXEMPLE 5

### Comprimé de chlorhydrate de dronédarone

On a préparé des comprimés de chlorhydrate de dronédarone de formulation identique à celle de l'Exemple 4, par application du procédé ci-dessous :

On mélange, après calibrage, 724,2g de chlorydrate de dronédarone, 110,5g de cellulose microcristalline, 2,2g de silice colloïdale anhydre, 72,5g de lactose anhydre, 22,1g de polyvinylpyrrolidone, 68g de poloxamère 407 fondu (SYNPERONIC® PE/F127) et 97,8g de macrogol 6000 fondu.

On poursuit ensuite de la même manière qu'à l'Exemple 4 pour obtenir des comprimés d'un poids de 650 mg par unité.

### EXEMPLE 6

### Comprimé de chlorhydrate de dronédarone

On a préparé des comprimés de chlorhydrate de dronédarone de formulation identique à celle de l'Exemple 4, toutefois après remplacement du macrogol 6000 par une quantité équivalente de poloxamère 407, et ce, par application du procédé ci-dessous :

On mélange après calibrage, 724,2g de chlorhydrate de dronédarone, 110,5g de cellulose microcristalline, 2,2g de silice colloïdale anhydre, 72,5g de lactose anhydre, 22,1g de polyvinylpyrrolidone et 166,7g de poloxamère 407 (SYNPERONIC® PE/F127).

On poursuit ensuite de la même manière qu'à l'Exemple 4 pour obtenir des comprimés d'un poids de 650mg par unité.

### EXEMPLES 7 et 8

En suivant les procédés décrits précédemment, on a préparé des comprimés de formulation suivante :

| a) | |
|---|---|
| Ingrédients | mg |
| Chlorhydrate de dronédarone (correspondant à 400mg de base) | 426 |
| Cellulose microcristalline | 26 |
| Amidon de maïs | 45,5 |
| Polyvinylpyrrolidone | 65 |
| Poloxamère 407 | 40 |
| Silice colloïdale anhydre | 2,6 |
| Stéarate de magnésium | 3,25 |
| Lactose monohydraté | 41,65 |
| | 650 |

| b) | |
|---|---|
| Ingrédients | mg |
| Chlorhydrate de dronédarone (correspondant à 400mg de base) | 213 |
| Cellulose microcristalline | 13 |
| Amidon de maïs | 22,75 |
| Polyvinylpyrrolidone | 32,5 |
| Poloxamère 407 | 20 |
| Silice colloïdale anhydre | 1,3 |
| Stéarate de magnésium | 1,625 |
| Lactose monohydraté | 20,825 |
| | 325 |

## Revendications

1. Composition pharmaceutique solide comprenant un dérivé de benzofuranne à activité antiarythmique adapté pour l'administration orale, **caractérisée en ce qu'**elle comprend un dérivé de benzofuranne à activité antiarythmique ou un de ses sels pharmaceutiquement acceptables, comme principe actif, et un agent tensioactif hydrophile non ionique pharmaceutiquement acceptable, en association avec un ou plusieurs excipients pharmaceutiques, et **en ce que** le dérivé de benzofuranne à activité antiarythmique est la dronédarone ou un de ses sels pharmaceutiquement acceptables.

2. Composition pharmaceutique selon la Revendication 1, **caractérisée en ce que** le sel pharmaceutiquement acceptable est le chlorhydrate.

3. Composition pharmaceutique selon une des Revendications 1 à 2, **caractérisée en ce que** l'agent tensioactif hydrophile non ionique est choisi parmi des poloxamères, des huiles de ricin polyéthoxylées, des polysorbates éthoxylés et des hydroxystéarates de polyéthylène.

4. Composition pharmaceutique selon la Revendication 3, **caractérisée en ce que** l'agent tensioactif hydrophile non ionique est choisi parmi les poloxamère 124, poloxamère 188, poloxamère 237, poloxamère 338, poloxamère 407, polysorbate 20, polysorbate 40, polysorbate 60, polysorbate 80 et le produits CREMOPHOR® RH 40 ou SOLUTOL ® HS15.

5. Composition pharmaceutique selon la Revendication 3 ou 4, **caractérisée en ce que** l'agent tensioactif hydrophile non ionisé est le poloxamère 407.

6. Composition pharmaceutique selon une des Revendications 1 à 5, **caractérisée en ce que** l'agent hydrophile non ionique est présent à raison de 1% à 50% en poids du principe actif sous forme de base.

7. Composition pharmaceutique selon la Revendication 6 sous forme de comprimé ou de gélule, **caractérisée en ce que** l'agent tensioactif hydrophile non ionique est présent à raison de 1 % à 20% en poids du principe actif sous forme de base.

8. Composition pharmaceutique selon la Revendication 7, sous forme de comprimé ou de gélule, **caractérisée en ce que** l'agent tensioactif hydrophile non ionique est présent à raison de 5% à 15% en poids du principe actif sous forme de base.

9. Composition pharmaceutique selon, une des Revendications 1 à 8, **caractérisée en ce qu'**elle contient de 50 à 500mg de principe actif par unité d'administration.

10. Composition pharmaceutique selon la Revendication 9, sous forme de comprimé ou de gélule, **caractérisée en ce qu'**elle contient, de 200 à 400mg de principe actif par unité d'administration.

11. Composition pharmaceutique selon une des Revendications 1 à 10 sous forme de comprimé ou de gélule, **caractérisée en ce qu'**elle contient, par unité d'administration, de 200 à 400mg de principe actif calculés sous forme de base et 10% en poids d'agent tensioactif hydrophile non ionique par rapport au principe actif sous forme de base.

## Patentansprüche

1. Feste pharmazeutische Zusammensetzung umfassend ein Benzofuranderivat mit antiarrhythmischer Wirkung, welche an die Verabreichung auf oralem Wege angepasst ist, **dadurch gekennzeichnet, dass** sie ein Benzofuranderivat mit antiarrhythmischer Wirkung oder eines seiner pharmazeutisch annehmbaren Salze als Wirkstoff und ein hydrophiles, nichtionisches, pharmazeutisch annehmbares oberflächenaktives Mittel in Kombination mit einem oder mehreren pharmazeutischen Trägermaterialien umfasst, und dass das Benzofuranderivat mit antiarrhythmischer Wirkung Dronedaron oder eines seiner pharmazeutisch annehmbaren Salze ist.

2. Pharmazeutische Zusammensetzung nach Anspruch 1, **dadurch gekennzeichnet, dass** das pharmazeutisch annehmbare Salz das Hydrochlorid ist.

3. Pharmazeutische Zusammensetzung nach einem der Ansprüche 1 bis 2, **dadurch gekennzeichnet, dass** das hydrophile, nichtionische oberflächenaktive Mittel ausgewählt ist aus Poloxameren, polyethoxylierten Rizinusölen, ethoxylierten Polysorbaten und Polyethylen-hydroxystearaten.

4. Pharmazeutische Zusammensetzung nach Anspruch 3, **dadurch gekennzeichnet, dass** das hydrophile, nichtionische oberflächenaktive Mittel ausgewählt ist aus Poloxamer 124, Poloxamer 188, Poloxamer 237, Poloxamer 338, Poloxamer 407, Polysorbat 20, Polysorbat 40, Polysorbat 60, Polysorbat 80 und den Produkten CREMOPHOR® RH 40 oder SOLUTOL® HS15.

5. Pharmazeutische Zusammensetzung nach Anspruch 3 oder 4, **dadurch gekennzeichnet, dass** das hydrophile, nichtionisierte oberflächenaktive Mittel Poloxamer 407 ist.

6. Pharmazeutische Zusammensetzung nach einem der Ansprüche 1 bis 5, **dadurch gekennzeichnet, dass** das hydrophile, nichtionische Mittel in einer Menge von 1 % bis 50 %, bezogen auf das Gewicht des Wirkstoffs in Form der Base, vorhanden ist.

7. Pharmazeutische Zusammensetzung nach Anspruch 6 in Form von Tabletten oder Gelkapseln, **dadurch gekennzeichnet, dass** das hydrophile, nichtionische oberflächenaktive Mittel in einer Menge von 1 % bis 20 %, bezogen auf das Gewicht des Wirkstoffs in Form der Base, vorhanden ist.

8. Pharmazeutische Zusammensetzung nach Anspruch 7 in Form von Tabletten oder Gelkapseln, **dadurch gekennzeichnet, dass** das hydrophile, nichtionische oberflächenaktive Mittel in einer Menge von 5 % bis 15 %, bezogen auf das Gewicht des Wirkstoffs in Form der Base, vorhanden ist.

9. Pharmazeutische Zusammensetzung nach einem der Ansprüche 1 bis 8, **dadurch gekennzeichnet, dass** sie 50 bis 500 mg des Wirkstoffs pro Verabreichungseinheit enthält.

10. Pharmazeutische Zusammensetzung nach Anspruch 9 in Form von Tabletten oder Gelkapseln, **dadurch gekennzeichnet, dass** sie 200 bis 400 mg des Wirkstoffs pro Verabreichungseinheit enthält.

11. Pharmazeutische Zusammensetzung nach einem der Ansprüche 1 bis 10 in Form von Tabletten oder Gelkapseln, **dadurch gekennzeichnet, dass** sie pro Verabreichungseinheit 200 bis 400 mg des Wirkstoffs, berechnet in Form der Base, und 10 Gew.-% des hydrophilen, nichtionischen oberflächenaktiven Mittels, bezogen auf den Wirkstoff in Form der Base, enthält.

## Claims

1. Solid pharmaceutical composition comprising a benzofuran derivative with antiarrhythmic activity and suitable for oral administration, **characterized in that** it comprises a benzofuran derivative with antiarrhythmic activity, or one of the pharmaceutically acceptable salts thereof, as an active principle, and a pharmaceutically acceptable nonionic hydrophilic surfactant, in combination with one or more pharmaceutical excipients, and **in that** the benzofuran derivative with antiarrhythmic activity is dronedarone or one of the pharmaceutically acceptable salts thereof.

2. Pharmaceutical composition according to Claim 1, **characterized in that** the pharmaceutically acceptable salt is the hydrochloride.

3. Pharmaceutical composition according to either of Claims 1 and 2, **characterized in that** the nonionic hydrophilic surfactant is chosen from poloxamers, polyethoxylated castor oils, ethoxylated polysorbates and polyethylene hydroxystearates.

4. Pharmaceutical composition according to Claim 3, **characterized in that** the nonionic hydrophilic surfactant is chosen from poloxamer 124, poloxamer 188, poloxamer 237, poloxamer 338, poloxamer 407, polysorbate 20, polysorbate 40, polysorbate 60, polysorbate 80 and the products Cremophor® RH 40 or Solutol® HS15.

5. Pharmaceutical composition according to Claim 3 or 4, **characterized in that** the nonionized hydrophilic surfactant is poloxamer 407.

6. Pharmaceutical composition according to one of Claims 1 to 5, **characterized in that** the nonionic hydrophilic agent is present in a proportion of from 1% to 50% by weight of the active principle in base form.

7. Pharmaceutical composition according to Claim 6, in tablet or gelatin capsule form, **characterized in that** the nonionic hydrophilic surfactant is present in a proportion of from 1% to 20% by weight of the active principle in base form.

8. Pharmaceutical composition according to Claim 7, in tablet or gelatin capsule form, **characterized in that** the nonionic hydrophilic surfactant is present in a proportion of from 5% to 15% by weight of the active principle in base form.

9. Pharmaceutical composition according to one of Claims 1 to 8, **characterized in that** it contains from 50 to 500 mg of active principle per administration unit.

10. Pharmaceutical composition according to Claim 9, in tablet or gelatin capsule form, **characterized in that** it contains from 200 to 400 mg of active principle per administration unit.

11. Pharmaceutical composition according to one of Claims 1 to 10, in tablet or gelatin capsule form, **characterized in that** it contains, per administration unit, from 200 to 400 mg of active principle, calculated in base form, and 10% by weight of nonionic hydrophilic surfactant relative to the active principle in base form.
